Europäisches Patentamt

European Patent Office

Office européen des brevets

(11) Veröffentlichungsnummer: **0 009 092**
**B1**

# (12) EUROPÄISCHE PATENTSCHRIFT

(45) Veröffentlichungstag der Patentschrift:
05.08.81

(21) Anmeldenummer: **79102565.3**

(22) Anmeldetag: **20.07.79**

(51) Int. Cl.³: **C 07 C 143/66**

(54) **Verfahren zur Herstellung von 1-Amino-8-naphthol-3,6-disulfonsäure (H-Säure).**

(30) Priorität: **04.08.78 DE 2834166**

(43) Veröffentlichungstag der Anmeldung:
**02.04.80 Patentblatt 80/7**

(45) Bekanntmachung des Hinweises auf die Patenterteilung:
**05.08.81 Patentblatt 81/31**

(84) Benannte Vertragsstaaten:
**BE CH DE FR GB IT**

(56) Entgegenhaltungen:
**DE-A1-2 716 030**
**DE-A1-2 727 345**

(73) Patentinhaber: **BAYER AG, Zentralbereich Patente, Marken und Lizenzen, D-5090 Leverkusen 1, Bayerwerk (DE)**

(72) Erfinder: **Behre, Horst, Dr., Im Hellsiefen 4, D-5068 Odenthal (DE)**
Erfinder: **Hullen, Albert, Opladener Strasse 216, D-4018 Langenfeld (DE)**
Erfinder: **Krüger, Bruno, Dr., Haferkamp 1, D-5000 Köln 80 (DE)**
Erfinder: **Steffan, Guido, Dr., Im Herzogenfeld 52, D-5068 Odenthal (DE)**

ACTORUM AG.

Verfahren zur Herstellung von 1-Amino-8-naphthol-3,6-disulfonsäure (H-Säure)

Die vorliegende Erfindung betrifft ein Verfahren zur Herstellung von 1-Amino-8-naphthol-3,6-disulfonsäure (H-Säure) als Monoalkalisalz aus Naphthylamin-trisulfonsäure-Isomerengemischen durch alkalische Druckhydrolyse.

1-Amino-8-naphthol-3,6-disulfonsäure, die häufig auch als H-Säure bezeichnet wird, ist ein wichtiges Zwischenprodukt zur Herstellung von Farbstoffen (s. Ullmanns Enzyklopädie der Technischen Chemie, 3. Auflage, 12. Band, S. 621).

Aus FIAT Final Report Nr. 1016, S. 32 bis 39, ist bekannt, dass man H-Säure wie folgt herstellen kann: Naphthalin wird mit Schwefelsäuremonohydrat (= 100 %iger $H_2SO_4$) und 65 %igem Oleum unter Einhaltung eines bestimmten Temperaturprogrammes und abgestufter Zugabe von Schwefelsäuremonohydrat und Oleum zu einem Naphthalin-trisulfonsäure-Isomerengemisch umgesetzt, das mit Mischsäure nitriert wird. Nach Verdünnung mit Wasser, Austreiben der nitrosen Gase und Abtrennung der Schwefelsäure als Calciumsulfat wird das Isomerengemisch der Nitro-Naphthalin-trisulfonsäuren mit Eisen reduziert und dann gelöste Eisensalze mit Magnesiumoxid gefällt und abgetrennt . Durch Zugabe von Steinsalz und Salzsäure wird das saure Calcium-Natrium-Salz der T-Säure (1-Naphthylamin-3,6,8-trisulfonsäure) ausgefällt, das abfiltriert und mehrmals gewaschen wird. Dieses Salz wird in Waschwasser eingetragen und mit Soda versetzt. Dann wird von der ausgefallenen Kreide abgepresst und die Salzlösung eingeengt. Die eingeengte Trinatriumsalzlösung der T-Säure wird mit 50 %iger Natronlauge unter Druck umgesetzt. Anschliessend wird die H-Säure aus dem alkalischen Hydrolysegemisch durch Ansäuern mit einem Gemisch aus Schwefelsäure und H-Säure-haltiger Waschbrühe vom vorhergehenden Ansatz als Mononatriumsalz ausgefällt, dann abfiltriert und, nach dem Waschen mit Wasser, getrocknet.

Bei diesem Verfahren ist nachteilig, dass auf einer Zwischenstufe die T-Säure als saures Calcium-Natrium-Salz abgetrennt werden muss, wobei ein Abwasser anfällt, dessen Aufarbeitung äusserst schwierig und kostspielig ist, da es neben organischen Bestandteilen auch grosse Mengen Natriumchlorid, Calciumchlorid und Salzsäure enthält. Das so abgeschiedene T-Säure-Salz enthält noch Natriumchlorid. Dieses kann nur unter Verlust von T-Säure ausgewaschen werden. Wenn man es in der T-Säure belässt, kann man die Lösung nach Abtrennung der Kreide nicht weiter einengen als bis zu einer Konzentration, die bei Titration von 100 g Lösung einem Nitritverbrauch von ca. 5,5 g entspricht, da sonst Salzablagerungen auftreten, die eine kontinuierliche Arbeitsweise nicht zulassen. Für die anschliessende Umsetzung mit Natronlauge ist jedoch eine möglichst konzentrierte Lösung erwünscht, um die zur Einstellung einer bestimmten Laugenkonzentration im Reaktionsgemisch benötigte Laugenmenge möglichst gering zu halten.

Aus der DE-OS 2 727 345 ist bekannt, die 1-Amino-8-naphthol-3,6-disulfonsäure (H-Säure) folgendermassen herzustellen: Ein 1-Nitronaphthalin-3,6,8-trisulfonsäure-Reaktionsgemisch, erhalten durch Trisulfierung von Naphthalin und anschliessender Nitrierung wird reduziert, die 1-Naphthylamin-3,6,8-trisulfonsäure wird isoliert, der Alkalischmelze unterworfen und aus dem erhaltenen Alkalischmelzgemisch wird die 1-Amino-8-naphthol-3,6-disulfonsäure als Monoalkalisalz durch Mischen mit dem 1-Nitro-naphthalin-3,6,8-trisulfonsäure-Reaktionsgemisch ausgefällt und durch Filtrieren abgetrennt. Das die 1-Nitronaphthalin-3,6,8-trisulfonsäure enthaltende Filtrat wird als Ausgangsmaterial für die Reduktionsreaktion verwendet.

Bei diesem Verfahren ist nachteilig, dass das gesamte, stark verdünnte Filtrat aus der Isolierung des Monoalkalisalzes der 1-Amino-8-naphthol-3,6-disulfonsäure, das neben der 1-Nitronaphthalin-3,6,8-trisulfonsäure die gesamten organischen Nebenprodukte und grosse Mengen Alkalisulfat enthält, in die Reduktionsreaktion mit Eisen oder anderen Reduktionsmitteln und die nachfolgende Ausfällung und Filtration der 1-Naphthylamin-3,6,8-trisulfonsäure als saures Alkalisalz eingesetzt werden muss. Daraus resultiert eine geringe Raum/Zeit-Ausbeute. Ausserdem wird die alkaliempfindliche 1-Nitronaphthalin-3,6,8-trisulfonsäure bei der Mischung mit dem Alkalischmelzgemisch hohen örtlichen Alkalikonzentrationen ausgesetzt. Daraus resultiert für das Gesamtverfahren im Endeffekt nur eine unbefriedigende Ausbeute an 1-Amino-8-naphthol-3,6-disulfonsäure, bezogen auf eingesetztes Naphthalin.

In dieser Anmeldung wird darauf hingewiesen, dass sich allein die als Fällungsmittel beanspruchten Nitro-T-Säure-Lösungen zum Ansäuern der alkalischen H-Säure-Lösungen bewährt haben, während andere Fremdstoffe enthaltende Schwefelsäure-Lösungen sich als ungeeignet erwiesen.

Überraschenderweise wurde nun gefunden, dass es bestimmte bei der Produktion von Farbstoff-Zwischenprodukten anfallende Abfallschwefelsäure-Lösungen gibt, die sich zum Ansäuern der alkalischen H-Säure-Lösungen eignen (von über 65 Gew.-% 1-Naphthylamin-3,6,8-trisulfonsäure enthaltenden).

Die Erfindung betrifft daher ein Verfahren zur Herstellung von 1-Amino-8-naphthol-3,6-disulfonsäure-Monoalkalisalzen durch Umsetzung von Naphthylamin-trisulfonsäure-Isomerengemischen und/oder deren Salzen mit Alkalihydroxidlösungen bei erhöhtem Druck und erhöhter Temperatur und anschliessendem Abscheiden der 1-Amino-8-naphthol-3,6-disulfonsäure in Form des Monoalkalisalzes durch Ansäuern, das dadurch gekennzeichnet ist, dass man zum Ansäuern 30 bis 60 Gew.-%-ige Abfallschwefelsäuren, bezogen auf $H_2SO_4$ und Wasser, aus der 2-Nitronaphthalin-4,8-disulfonsäure-Magnesiumsalz-Fällung, der 2-Naphthylamin-5,7-disulfonsäure-Fällung und/oder der Naphthalin-1,5-disulfonsäure-Fällung verwendet, wobei die Abfallschwefelsäure aus der 2-Nitronaphthalin-4,8-disulfonsäure-Magnesiumsalz-Fällung 1 bis 10 Gew.-% Naphthalin- und Nitronaphthalin-mono-, -di- und -trisulfonsäuren, die Abfallschwefelsäure aus der 2-Naphthylamin-5,7-disulfonsäure-Fällung 1 bis 6 Gew.-% Naphthylamin-mono-, -di- und -trisulfonsäuren und die Abfallschwefelsäure aus der Naphthalin-1,5-disulfonsäure-Fällung 10 bis 30 Gew.-% Naphthalin-mono-, -di- und trisulfonsäuren und gegebenenfalls Natriumionen enthält.

In das erfindungsgemässe Verfahren einsetzbare Naphthyl-amintrisulfonsäure-Isomerengemische wie z.B. bei der technischen Herstellung von T-Säure anfallen, enthalten, bezogen auf die Gesamtmenge diazotierbarer Substanzen über 65 Gew.-% 1-Naphthylamin-3,6,8-trisulfonsäure (T-Säure). Bevorzugt werden solche Gemische eingesetzt, die 70 bis 90 Gew.-% 1-Naphthylamin-3,6,8-trisulfonsäure enthalten. Ein besonders bevorzugt einzusetzendes Naphthylamintrisulfonsäure-Gemisch enthält

75 bis 85 Gew.-% 1-Naphthylamin-3,6,8-trisulfonsäure

5 bis 15 Gew.-% 1-Naphthylamin-3,5,7-trisulfonsäure

1 bis 10 Gew.-% 1-Naphthylamin-4,6,8-trisulfonsäure

0,5 bis 5 Gew.-% 1-Naphthylamin-2,5,7-trisulfonsäure

0,1 bis 2 Gew.-% 2-Naphthylamin-3,5,7-trisulfonsäure

0,1 bis 2 Gew.-% 2-Naphthylamin-4,6,8-trisulfonsäure

0,1 bis 2 Gew.-% 2-Naphthylamin-3,6,8-trisulfonsäure

Solche Gemische können beispielsweise erhalten werden, indem man Naphtalin trisulfiert, das entstandene Gemisch nitriert und das dann vorliegende Nitro-Naphthalintrisulfonsäure-Gemisch reduziert. Diese Reaktionen können gemäss der eingangs beschriebenen Arbeitsweise nach FIAT Final Report Nr. 16, S. 37 bis 38, oder auf beliebige andere Weise durchgeführt werden.

Das Naphthylamintrisulfonsäure-Gemisch kann die Säuren in freier Form, in Form von neutralen Salzen oder in Form von sauren Salzen enthalten. Auch Gemische, die freie Säuren und Salze enthalten, können verwendet werden. Sofern die Naphthylamintrisulfonsäuren ganz oder teilweise als Salze vorliegen, sind die Alkali- und Erdalkalisalze, insbesondere die Natrium- und Kaliumsalze, bevorzugt. Ganz besonders bevorzugt sind Naphthylamintrisulfonsäure-Gemische, welche die Säuren in Form von Trinatriumsalzen enthalten.

Das Naphthylamintrisulfonsäuregemisch kann neben den Naphthylamintrisulfonsäuren bzw. deren Salzen noch weitere Produkte enthalten. Solche Produkte können insbesondere Nebenprodukte, Zersetzungsprodukte oder nicht umgesetzte Zwischenprodukte aus den Herstellungsstufen für Naphthylamintrisulfonsäure sein, beispielsweise Naphthalin-di-,-tri- und -tetra-sulfonsäuren, Nitronaphthalin-mono-, -di- und -trisulfonsäuren, Naphthylamin-mono- und -disulfonsäuren, z.B. 1-Naphthylamin-3,6- und -5,7-disulfonsäure, weiterhin Dinaphthylsulfon-sulfosäuren und deren Amino- und Nitroderivate, sowie Oxydationsprodukte des Naphthalins und/oder der Naphthalinsulfonsäuren, die bei der Sulfonierung und/oder der Nitrierung gebildet werden können.

Das Naphthylamintrisulfonsäure-Gemisch kann in fester Form oder als wässrige Lösung mit einem Gehalt von beispielsweise 20 bis 50 Gew.-%, vorzugsweise 30 bis 40 Gew.-%, gerechnet als freie Säure mit dem Molekulargewicht 383, eingesetzt werden.

Als Alkalihydroxidlösungen für das erfindungsgemässe Verfahren kommen insbesondere wässrige Kali- oder Natronlauge in Frage. Der Einsatz von Kalilauge führt im Vergleich zu Natronlauge zu besseren Ausbeuten, Natronlauge ist jedoch im allgemeinen kostengünstiger. Pro Mol diazotierbare Substanz (gerechnet mit Molekulargewicht 383 = T-Säure) können beispielsweise 2,5 bis 12 Mol Alkalihydroxid eingesetzt werden. Besonders bevorzugt ist der Einsatz von 6 bis 9 Mol Alkalihydroxid pro Mol diazotierbare Substanz. Die Konzentration von Alkalihydroxid im Reaktionsgemisch kann beispielsweise 10 bis 50 Gew.-% (bezogen auf die Summe Alkalihydroxid + gesamtes Wasser) betragen. Bevorzugt ist diese Konzentration 25 bis 35 Gew.-%ig.

Die Umsetzung kann bei Temperaturen von 150 bis 250°C, vorzugsweise bei 180 bis 220°C in einem geschlossenen Gefäss durchgeführt werden. Der sich dabei einstellende Druck ist im allgemeinen völlig ausreichend, um das erfindungsgemässe Verfahren in befriedigender Weise durchzuführen. Selbstverständlich kann man das erfindungsgemässe Verfahren auch bei anderen Drücken durchführen als denjenigen, die sich im geschlossenen Gefäss von selbst einstellen. Beispielsweise sind für das erfindungsgemässe Verfahren Drücke im Bereich von 5 bis 50 bar möglich.

Die Reaktionszeit hängt im wesentlichen von der Reaktionstemperatur und der Alkalihydroxid-Konzentration ab. Sie ist bei relativ hohen Reaktionstemperaturen und bei relativ hohen Alkalihydroxid-Konzentrationen kürzer und bei relativ niedrigen Reaktionstemperaturen und relativ niedrigen Alkalihydroxid-Konzentrationen länger und beträgt im allgemeinen 10 Minuten bis 10 Stunden. Beispielsweise werden bei einer Reaktionstemperatur von ca. 200°C und einer Alkalihydroxid-Konzentration von 30 Gew.-% mit einer Reaktionszeit von einer Stunde gute Ergebnisse erhalten.

Bei der Durchführung des Verfahrens ist es günstig, wenn während der gesamten Reaktion günstige Alkalihydroxid-Konzentration vorliegen. Man arbeitet deshalb vorzugsweise so, dass man das Naphthylamintrisulfonsäure-Isomerengemisch und die Hauptmenge der Alkalihydroxidlösung simultan im Verlaufe von beispielsweise 5 bis 30 Minuten, vorzugsweise 10 bis 20 Minuten, in eine Vorlage aus wenig Alkalihydroxidlösung einpumpt und das Gemisch anschliessend ausreagieren lässt. Die Ausgangsstoffe werden am zweckmässigsten mit einer solchen Temperatur in das Reaktionsgefäss eingebracht, dass man nach Freiwerden der Mischungs- und gegebenenfalls der Neutralisationswärme die gewünschte Reaktionstemperatur vorliegen hat. Man kann die Ausgangsstoffe auch bei tieferen Temperaturen zusammenbringen und im Reaktionsgefäss auf die gewünschte Reaktionstemperatur erhitzen.

Nach Beendigung der Reaktion und vor Abscheiden der H-Säure als Monoalkalisalz ist es vorteilhaft, das Reaktionsgemisch zu kühlen und/oder mit Wasser zu verdünnen. Man kann beispielsweise auf Temperaturen im Bereich 20 bis 150°C, vorzugsweise auf Temperaturen im Bereich 80 bis 120°C, abkühlen. Die gegebenenfalls zuzusetzende Wassermenge richtet sich nach den Reaktionsbedingungen, z.B. der Art des Alkalihydroxids, dessen Menge und Konzentration. Es ist vorteilhaft, die Wassermenge so zu wählen, dass das bei der Reaktion gebildete Alkalisulfit gelöst wird.

Die erfindungsgemäss zur Abscheidung der H-Säure als Monoalkalisalz zu verwendenden wässrigen Abfall-Schwefelsäuren enthalten 30 bis 60 Gew.-% $H_2SO_4$, bezogen auf $H_2SO_4$ und Wasser. Diese Abfallschwefelsäuren fallen bei der technischen Herstellung von 2-Nitronaphthalin-4,8-disulfonsäure-Magnesiumsalz, bei der technischen Herstellung von 2-Naphthylamin-5,7-disulfonsäure und bei der technischen Herstellung von Naphthalin-1,5-disulfonsäure als freie Säure oder als Dinatriumsalz an. Dabei kann die Herstellung der 2-Nitronaphthalin-4,8-disulfonsäure z.B. nach dem in Ullmann, Enzyklopädie der Technischen Chemie, 3. Auflage, Band 9, Seite 629, oder nach dem in der US-PS 17 56 537 beschriebenen Verfahren oder nach beliebigen anderen Verfahren erfolgen. Die bei der technischen Herstellung von 2-Nitronaphthalin-4,8-disulfonsäure-magnesiumsalz anfallenden Abfallschwefelsäuren enthalten insgesamt 1–10 Gew.% Naphthalin- und Nitronaphthalin-mono-, -di- und -trisulfonsäuren. Die technische Herstellung der 2-Naphthylamin-5,7-disulfonsäure kann z.B. nach dem in Ullmann, Enzyklopädie der Technischen Chemie, 3. Auflage, Band 9, S. 629, beschriebenen Verfahren erfolgen. Die bei diesem Verfahren anfallenden Abfallschwefelsäuren enthalten insgesamt 1 bis 6 Gew.-% Naphthylamin-mono-, -di- und -trisulfonsäuren. Die bei der technischen Herstellung von Naphthalin-1,5-disulfonsäure als freie Säure oder als Dinatriumsalz anfallenden Abfallschwefelsäuren können sowohl bei der Herstellung von Naphthylamin-1,5-disulfonsäure gemäss dem in FIAT Final Report Nr. 1016, S. 45 – 47, beschriebenen Verfahren als auch bei anderen Herstellungsverfahren für Naphthalin-1,5-disulfonsäure anfallen. Die bei der technischen Herstellung von Naphthalin-1,5-disulfonsäure anfallenden Abfallschwefelsäuren enthalten 10 bis 30 Gew.-% Naphthalin-mono-, -di- und -trisulfonsäuren und gegebenenfalls Natriumionen.

Die erfindungsgemäss zu verwendenden Abfallschwefelsäuren können einzeln oder in beliebigen Mischungen miteinander zur erfindungsgemässen Abscheidung der H-Säure verwendet werden.

Es ist vorteilhaft, die Abfallschwefelsäuren vor ihrer Verwendung zum Ausfällen der H-Säure zu filtrieren und/oder mit Wasser und/oder mit Frischschwefelsäure zu versetzen.

Zur Vermeidung von Ausbeuteverlusten bei der H-Säure-Abscheidung und zur Erzielung einer sehr guten H-Säure-Qualität ist es vorteilhaft, bei jeder Abfallschwefelsäure bzw. Abfallschwefelsäure-Mischung getrennt zu ermitteln mit welchen Zusätzen an Frischschwefelsäure und gegebenenfalls Wasser die besten Ergebnisse erhalten werden.

Beispielsweise wurden im allgemeinen gute Ergebnisse erhalten, wenn man pro Mol Abfallschwefelsäure aus der 2-Nitronaphthalin-4,8-disulfonsäure-Magnesiumsalz-Herstellung mindestens 0,25 Mol Frisch-Schwefelsäure zusetzt. Bei der Verwendung von Abfallschwefelsäure aus der 2-Naphthylamin-5,7-disulfonsäure-Herstellung ist es im allgemeinen zur Erzielung guter Ergebnisse nicht erforderlich, Frischschwefelsäure zuzusetzen, ein Frischschwefelsäurezusatz hat andererseits keine technisch nachteiligen Folgen.

Bei Verwendung von Abfallschwefelsäure/Frischschwefelsäure-Gemischen ist es im allgemeinen nicht erforderlich, mehr als 1 Mol Frischschwefelsäure pro Mol Abfallschwefelsäure einzusetzen. Die Konzentration der Frischschwefelsäure kann beliebig gewählt werden, beispielsweise zwischen 20 und 80 Gew.-%. Vorzugsweise wird 40 bis 60 gew.-%ige Frischschwefelsäure verwendet.

Die Konzentration der Schwefelsäure oder des Schwefelsäuregemisches, das zum Ansäuern zur Abscheidung von 1-Amino-8-naphthol-3,6-disulfonsäure als Monoalkalisalz verwendet wird, kann ebenfalls in einem weiten Bereich variiert werden; beispielsweise zwischen 20 und 80 Gew.-%. Vorzugsweise liegt diese Konzentration im Bereich 30 bis 60 Gew.-%. Diese Konzentrationen können z. B. durch Zugabe von Wasser und/oder Frischschwefelsäure geeigneter Konzentration variiert werden.

Zur Abscheidung der H-Säure gibt man soviel Abfallschwefelsäure und/oder Abfallschwefelsäure/Frischschwefelsäure-Gemisch zu, dass sich das schwerlösliche Monoalkalisalz der H-Säure bildet. Durch entsprechende Wahl der Konzentration der Abfallschwefelsäure und/oder durch Zugabe von Wasser vor und/oder während der Zugabe der Abfallschwefelsäure wird zweckmässigerweise dafür gesorgt, dass das sich bildende anorganische Salz, z.B. Natriumsulfat oder Kaliumsulfat, nicht ausfällt. Man kann beispielsweise gute Ergebnisse erhalten, wenn man zur Abscheidung der H-Säure als Monoalkalisalz einen pH-Wert im Bereich 0 bis 4, vorzugsweise 0,5 bis 2,5, einstellt und durch Verdünnung mit Wasser und/oder durch entsprechende Wahl der Konzentration der Abfall-Schwefelsäure, bezogen auf das Gewicht des in der Druckhydrolyse vorliegenden Gemisches, die 0,1- bis 5-fache, vorzugsweise die 0,5- bis 2-fache Menge Wasser einbringt. Die Abtrennung des Monoalkalisalzes der H-Säure kann auf übliche Weise, beispielsweise durch Filtration, erfolgen. Es ist vorteilhaft, vor der Abtrennung des Monoalkalisalzes der H-Säure die Temperatur durch Kühlung, beispielsweise durch Verdampfungskühlung auf weniger als 80°C einzustellen und die Abtrennung bei einer Temperatur von weniger als 80°C vorzunehmen. Bevorzugt wird die Abtrennung bei einer Temperatur im Bereich von 20 bis 60°C vorgenommen.

Zur vollständigen Entfernung von Schwefeldioxid ist es vorteilhaft, nach Einstellung der Fällungsbedingungen und vor der Abtrennung des Monoalkalisalzes der H-Säure das angesäuerte und verdünnte Gemisch für einige Zeit, beispielsweise 0,5 bis 2 Stunden, am Rückfluss zu kochen oder unter Vakuum zu halten oder das Schwefeldioxid mit einem inerten Gas, z.B. Stickstoff, auszublasen.

Das nach der Abtrennung vorliegende Monoalkalisalz der H-Säure wäscht man üblicherweise mit Wasser und trocknet es, beispielsweise im Vakuum.

Nach dem erfindungsgemässen Verfahren kann man das gebildete Monoalkalisalz der H-Säure in nahezu quantitativer Ausbeute und sehr hoher Reinheit (z.B. 98–99 % bezogen auf insgesamt vorliegende organische Verbindungen) isolieren.

Dies ist sehr überraschend, da sich schon in dem nach der Druckhydrolyse vorhandenen Gemisch ca. 25 verschiedene Komponenten nachweisen lassen und mit der eingesetzten Abfallschwefelsäure der Reaktionsmischung eine grössere Anzahl weiterer Nebenkomponenten zugeführt wird.

Das erfindungsgemässe Verfahren bietet gegenüber den bisher üblichen alkalischen Druckhydrolysen von abgeschiedener T-Säure und Abscheidung der H-Säure mit Frischschwefelsäure erhebliche Vorteile und ist daher besonders wirtschaftlich. So ergibt sich zum einen eine erhebliche Einsparung an Schwefelsäure. Zum anderen ergibt sich für die Gesamtfabrikation der sogenannten Buchstabensäuren (Farbstoffzwischenprodukte auf Naphthalinbasis) eine wesentliche Verringerung des technologisch schwer beherrschbaren Dünnsäureanfalls. Gegenüber dem bisherigen Verfahren sind die Gesamtabwasser- und Salzmengen sehr stark reduziert.

Beispiel 1

In einem 2,7 Liter Nickel-Autoklav werden 580 g eines Naphthylamintrisulfonsäure-Gemisches in Form der Trinatriumsalze (Gehalt 11,9 g Gesamtnitrit/100 g, 52,8 Gew.-% T-Säure MG 383; insgesamt 69 g Nitrit, 0,80 Mol T-Säure) folgender Zusammensetzung:

| | |
|---|---|
| 1-Naphthylamin-3,6,8-trisulfonsäure | 80,0% |
| 1-Naphthylamin-3,5,7-trisulfonsäure | 8,5% |
| 1-Naphthylamin-4,6,8-trisulfonsäure | 4,0% |
| 1-Naphthylamin-2,5,7-trisulfonsäure | 3,0% |
| 2-Naphthylamin-3,5,7-trisulfonsäure | 1,2% |
| 2-Naphthylamin-4,6,8-trisulfonsäure | 0,7% |
| 2-Naphthylamin-3,6,8-trisulfonsäure | 0,5% |

(%-Gehalte jeweils bezogen auf diazotierbare Substanz) das zusätzlich

0,3 Gew.-% 1-Naphthylamin-3,6-disulfonsäure-dinatriumsalz,

1,3 Gew.-% Naphtalin-1,3,6-trisulfonsäure-trinatriumsalz,

0,6 Gew.-% 1-Nitronaphthalin-3,6,8-trisulfonsäure-trinatriumsalz.

4,6 Gew.-% Wasser und quantitativ nicht bestimmbare Mengen an Amino- und Nitroderivaten der Dinaphthylsulfon-sulfosäuren und an Oxidationsprodukten des Naphtalins und der Naphthalin-trisulfonsäuren enthält und 400 g Wasser vorgelegt und auf 180°C aufgeheizt. In einem 1,3 Liter Stahl-Autoklav werden 600 g 50 Gew.-%ige Natronlauge (7,5 Mol NaOH) auf 185°C aufgeheizt und mit Stickstoff in den 2,7 Liter Autoklaven gedrückt, wodurch bezogen auf das gesamte Wasser eine 30 Gew.-%ige Natronlauge entsteht. Dabei stellt sich eine Temperatur von 200°C ein. Die Reaktionsmischung wird 45 Minuten bei 200°C gehalten, so schnell wie möglich auf 100°C abgekühlt und mit 400 g Wasser verdünnt. Die heisse Reaktionslösung wird pH-kontrolliert auf pH 1 bis 1,5 mit 1100 g einer Mischung aus einem Gewichtsteil 48 gew.-%iger $H_2SO_4$ und einem Gewichtsteil einer Abfallschwefelsäure aus der 2-Nitronaphthalin-4,8-disulfonsäure-Magnesiumsalz-Isolierung folgender Zusammensetzung:

| | | |
|---|---|---|
| 42 | Gew.-% $H_2SO_4$ | |
| 2,2 | Gew.-% Nitronaphthalin-di- und -trisulfonsäuren | |
| 2,2 | Gew.-% Naphthalin-di- und -trisulfonsäuren | |
| 0,25 | Gew.-% Magnesiumionen | |
| 2,6 | Gew.-% organisch gebundener Kohlenstoff | |
| 0,15 | Gew.-% organisch gebundener Stickstoff | |
| 1,9 | Gew.-% organisch gebundener Schwefel | |

angesäuert, zur vollständigen Entfernung von Schwefeldioxid eine Stunde unter Rückfluss erhitzt, unter Verdampfungskühlung auf 40°C abgekühlt und 2 Stunden bei 40°C gehalten. Das Produkt wird bei 40°C filtriert, mit insgesamt 500 g Wasser gewaschen und bei 80°C im Vakuum getrocknet.

Die Ausbeute beträgt 57%, bezogen auf T-Säure-Isomerengemisch bzw. 71,5% bezogen auf T-Säure. Die H-Säure-Qualität wurde durch Hochdruck-Flüssigkeits-Chromatographie wie folgt ermittelt:

| | |
|---|---|
| H-Säure-Mononatriumsalz | 88,2% |
| 1-Naphthylamin-3,6-disulfonsäure-Mononatriumsalz | 0,1% |
| W-Säure-Mononatriumsalz | unter 0,1% |

| | |
|---|---|
| Chromotrop-Säure-Dinatriumsalz | 1,1% |
| T-Säure-Dinatriumsalz | 0,2% |
| Wasser | 9,5% |
| Natriumsulfat | 0,4% |
| Magnesium | unter 5 ppm |

Reaktionsprodukte aus den isomeren Naphthylamintrisulfonsäuren und Nebenkomponenten aus der Abfallschwefelsäure konnten im isolierten Produkt nicht nachgewiesen werden.

Beispiel 2

Es wurde wie im Beispiel 1 verfahren, jedoch wurde die auf 100°C abgekühlte Reaktionsmischung mit 300 g Waser verdünnt und die Lösung pH-kontrolliert (pH 1 bis 1,5) mit 1200 g Abfallschwefelsäure aus der 2-Naphthylamin-5,7-disulfonsäure-Isolierung mit folgender Zusammensetzung:

41 Gew.-% $H_2SO_4$

3,2 Gew.-% Naphthylamin-di- und -trisulfonsäuren angesäuert.

Die Ausbeute beträgt 56% bezogen auf T-Säure-Isomerengemisch bzw. 70% bezogen auf T-Säure. Die H-Säure-Qualität wurde durch Hochdruck-Flüssigkeits-Chromatographie wie folgt ermittelt:

| | |
|---|---|
| H-Säure-Mononatriumsalz | 88,5% |
| W-Säure-Mononatriumsalz | 0% |
| 1-Naphthylamin-3,6-disulfonsäure-mononatriumsalz | 0,1 bis 0,2% |
| Chromotrop-Säure-Dinatriumsalz | 0,9 bis 1,1% |

| | |
|---|---|
| T-Säure-Dinatriumsalz | 0,1 bis 0,2% |
| Wasser | 9,5% |
| Natriumsulfat | 0,3% |

2-Naphthylamin-di- und -trisulfonsäuren aus der Abfallschwefelsäure liessen sich in dem isolierten Produkt nicht nachweisen.

Beispiel 3

Es wurde wie in Beispiel 1 verfahren, jedoch wurde die auf 100°C abgekühlte Reaktionsmischung mit 300 g Waser verdünnt und die Lösung pH-kontrolliert (pH 1 bis 1,5) mit 1200 g Abfallschwefelsäure aus der Naphthalin-1,5-disulfonsäure-Isolierung mit folgender Zusammensetzung:

33 Gew.-% $H_2SO_4$

22,4 Gew.-% Naphthalin-di- und -trisulfonsäuren angesäuert.

Es wurden die in Beispiel 2 angegebenen Ausbeuten und Gehalte erhalten.

Naphthalin-di- und -trisulfonsäuren aus der Abfallschwefelsäure lassen sich mittels Hochdruck-Flüssigkeitschromatographie in dem isolierten Produkt nicht nachweisen.

Beispiel 4 a-g

Es wurde wie in Beispiel 1 verfahren, jedoch wurde die auf 100°C abgekühlte und mit 400 g Wasser verdünnte alkalische Reaktionslösung mit Mischungen aus den verschiedenen Abfallschwefelsäuren der Beispiele 1 bis 3 und/oder Frisch-Schwefelsäure angesäuert. Die Ergebnisse sind in Tabelle 1 zusammengefasst.

Tabelle 1

| Bei-spiel | Fällungsmittel (jeweils Gewichtsteile) | | | | Ausbeute (g) | Qualität (jeweils %) | | | | |
|---|---|---|---|---|---|---|---|---|---|---|
| | 50 gew.-%ige Frisch-$H_2SO_4$ | Abfall-$H_2SO_4$ aus 2-Nitro-naphthalin-4,8-disul-fonsäure-Herstellung | Abfall-$H_2SO_4$ aus 2-Naphthy-lamin-5,7-disulfon-säure-Herstellung | Abfall-$H_2SO_4$ aus Naphthalin-1,5-disul-fonsäure-Herstellung | H-Säure (MG319) pro 1 kg Reaktions-lösung | H-Säure (MG319) | 1-Naph-thylamin-3,6-disul-fonsäure (MG303) | W-Säu-re (MG319) | Chromo-trop-Säure (MG320) | T-Säure (MG383) |
| 4a* | 1 | 0 | 0 | 0 | 93,4 | 80,3 | 0,1 | 0 | 1,0 | 0,2 |
| 4b | 2 | 1 | 0 | 0 | 93,8 | 80,8 | 0,1 | 0 | 1,1 | 0,2 |
| 4c | 1 | 2 | 0 | 0 | 91,6 | 79,4 | 0,1 | 0 | 1,4 | 0,2 |
| 4d | 1 | 0 | 1 | 0 | 93,3 | 81,3 | 0,2 | 0 | 0,9 | 0,1 |
| 4e | 2 | 1 | 1 | 0 | 92,9 | 80,4 | 0,2 | 0 | 1,0 | 0,1 |
| 4f | 1 | 0 | 0 | 1 | 93,4 | 79,6 | 0,1 | 0,2 | 1,1 | 0,2 |
| 4g | 1 | 1 | 1 | 1 | 92,7 | 80,1 | 0,2 | 0,2 | 1,2 | 0,2 |

* nicht erfindungsgemäss; zum Vergleich

**Patentansprüche**

1. Verfahren zur Herstellung von 1-Amino-8-naphthol-3,6-disulfonsäure-monoalkalisalzen (H-Säure-monoalkalisalzen) durch Umsetzung von über 65 Gew.-% 1-Naphthylamin-3,6,8-trisulfonsäure enthaltenden Naphthylamin-trisulfonsäure-Isomerengemischen und/oder deren Salzen mit Alkalihydroxidlösungen bei erhöhtem Druck und erhöhter Temperatur und anschliessendem Abscheiden der 1-Amino-8-naphthol-3,6,-disulfonsäure (H-Säure) in Form des Monoalkalisalzes durch Ansäuern, dadurch gekennzeichnet, dass man zum Ansäuern 30 bis 60 Gew.-

%ige Abfallschwefelsäuren, bezogen auf H₂SO₄ und Wasser, aus der 2-Nitronaphthalin-4,8-disulfonsäure-Magnesiumsalz-Fällung, der 2-Naphthylamin-5,7-disulfonsäure-Fällung und/oder der Naphthalin-1,5-disulfonsäure-Fällung verwendet, wobei die Abfallschwefelsäure aus der 2-Nitronaphthalin-4,8-disulfonsäure-Magnesiumsalz-Fälung 1 bis 10 Gew.-% Naphthalin- und Nitronaphthalinmono-, -di- und -trisulfonsäuren, die Abfallschwefelsäure aus der 2-Naphthylamin-5,7-disulfonsäure-Fällung 1 bis 6 Gew.-% Naphthylamin-mono-, -di- und -trisulfonsäuren und die Abfallschwefelsäure aus der Naphthalin-1,5-disulfonsäure-Fällung10 bis 30 Gew.-% Naphthalinmono-, -di- und -trisulfonsäuren und gegebenenfalls Natriumionen enthält.

2. Verfahren nach Anspruch 1, dadurch gekennzeichnet, dass die Abfallschwefelsäuren vor ihrer Verwendung zum Ausfällen der H-Säure filtriert und/oder mit Wasser und/oder mit Frischschwefelsäure versetzt worden sind.

3. Verfahren nach Ansprüchen 1 und 2, dadurch gekennzeichnet, dass man zur Abscheidung der H-Säure einen pH-Wert im Bereich von 0 bis 4 einstellt.

4. Verfahren nach Ansprüchen 1 bis 3, dadurch gekennzeichnet, dass man vor Abtrennung des Monoalkalisalzes der H-Säure auf Temperaturen von weniger als 80°C abkühlt.

## Claims

1. Process for the preparation of 1-amino-8-naphthol-3,6-disulphonic acid mono-alkali metal salts (H-acid mono-alkali metal salts) by reacting naphthylamine-trisulphonic acid isomer mixtures containing over 65% by weight of 1-naphthylamine-3,6,8-trisulphonic acid and/or their salts with alkali metal hydroxide solutions at an elevated pressure and elevated temperature and subsequently separating out the 1-amino-8-naphthol-3,6-disulphonic acid (H-acid) in the form of the mono-alkali metal salt by acidification, characterised in that 30 to 60% strength by weight, relative to H₂SO₄ and water, waste sulphuric acids from the precipitation of the magnesium salt of 2-nitronaphthalene-4,8-disulphonic acid, from the precipitation of 2-naphthylamine-5,7-disulphonic acid and/or from the precipitation of naphthalene-1,5-disulphonic acid are used for the acidification, the waste sulphuric acid from the precipitation of the magnesium salt of 2-nitronaphthalene-4,8-disulphonic acid containing 1 to 10% by weight of naphthalene and nitronaphthalene-mono-, -di- and -tri-sulphonic acids, the waste sulphuric acid from the precipitation of 2-naphthylamine-5,7-disulphonic acid containing 1 to 6% by weight of naphthylamine-mono-, -di- and -tri-sulphonic acids and the waste sulphuric acid from the precipitation of naphthalene-1,5-disulphonic acid containing 10 to 30% by weight of naphthalene-mono-, -di- and -tri-sulphonic acids and possibly sodium ions.

2. Process according to claim 1, characterised in that the waste sulphuric acids have been filtered and/or mixed with water and/or with fresh sulphuric acid before being used to precipitate the H-acid.

3. Process according to claims 1 and 2, characterised in that in order separate out the H-acid the pH is brought to a value in the range from 0 to 4.

4. Process according to claims 1 to 3, characterised in that before separating off the mono-alkali metal salt of H-acid the mixture is cooled to temperatures below 80°C.

## Revendications

1. Procédé pour la préparation de sels monoalcalins de l'acide 1-amino-8-naphtol-3,6-disulfonique (sels monoalcalins d'acide H) par réaction de mélanges d'acides naphtylaminetrisulfoniques isomères contenant plus de 65% en poids d'acide 1-naphtylamine-3,6,8 trisulfonique et/ou de leurs sels, avec des solutions d'hydroxydes alcalins sous pression élevée et à température élevée, puis séparation de l'acide 1-amino-8-naphtol-3,6-disulfonique (acide H) sous forme du sel monoalcalin par acidification, caractérisé en ce que l'on utilise pour l'acidification des acides sulfuriques résiduaires à 30–60% en poids, rapportés à H₂SO₄ et à l'eau, provenant de la précipitation du sel de magnésium d'acide 2-nitronaphtalène-4,8-di-sulfonique, de la précipitation de l'acide 2-naphtylamine-5,7-di-sulfonique et/ou de la précipitation de l'acide naphtalène-1,5-di-sulfonique, l'acide sulfonique résiduaire de la précipitation du sel de magnésium de l'acide 2-nitronaphtalène-4,8 disulfonique contenant 1 à 10% en poids d'acides naphtalène- et nitronaphtalène-mono-, di- et trisulfoniques, l'acide sulfurique résiduaire de la précipitation de l'acide naphtylamine-5,7 disulfonique contenant 1 à 6% en poids d'acides naphtylamine-mono-, di- et trisulfoniques et l'acide sulfurique résiduaire de la précipitation de l'acide naphtalène-1,5 disulfonique contenant 10 à 30% en poids d'acides naphtalène-mono-, di- et trisulfoniques et éventuellement des ions sodium.

2. Procédé selon la revendication 1, caractérisé en ce que l'on filtre les acides sulfuriques résiduaires et/ou on y ajoute de l'eau et/ou de l'acide sulfurique frais avant leur utilisation pour la précipitation de l'acide H.

3. Procédé selon les revendications 1 et 2, caractérisé en ce que l'on établit pour la précipitation de l'acide H une valeur de pH dans le domaine de 0 à 4.

4. Procédé selon les revendications 1 à 3, caractérisé en ce que l'on refroidit à des températures de moins de 80°C avant la séparation du sel monoalcalin de l'acide H.